# EUROPÄISCHE PATENTSCHRIFT

(11) **EP 1 551 280 B1**
(45) Veröffentlichungstag und Bekanntmachung des Hinweises auf die Patenterteilung: **19.11.2008**
(21) Anmeldenummer: 03753532.5
(22) Anmeldetag: 09.10.2003
(51) Int. Cl.: A61B 5/00, A61B 10/00, A61M 25/00

(54) **VERFAHREN ZUR HERSTELLUNG EINER MIKRODIALYSESONDE**
METHOD FOR THE PRODUCTION OF A MICRODIALYSIS PROBE
PROCEDE DE PRODUCTION D'UN SONDE DE MICRODIALYSE

(30) Priorität: 09.10.2002 DE 10247023
(43) Veröffentlichungstag der Anmeldung: 13.07.2005
(73) Patentinhaber: Disetronic Licensing AG, 3401 Burgdorf (CH)
(72) Erfinder: CARR, Matthew, Cambridge CB4 3EW (GB); REIHL, Bruno, CH-8832 Wilen bei Wollerau (CH); HEINIGER, Hanspeter, CH-4932 Lotzwil (CH); JOST, Stefan, CH-3203 Mühleberg (CH)
(74) Vertreter: Schalch, Rainer
(86) Internationale Anmeldenummer: PCT/EP2003/011182
(87) Internationale Veröffentlichungsnummer: WO 2004/032735

(56) Entgegenhaltungen:
- WO-A-00/10464
- WO-A-01/10483
- US-B1- 6 199 262
- US-B1- 6 478 767

## Beschreibung

Die Erfindung betrifft ein Verfahren zur Herstellung einer Mikrodialysesonde

In der Medizinaltechnik werden Mikrodialysesonden zum Beispiel zur Messung von Konzentrationen bestimmter gelöster Stoffe in Flüssigkeiten eines Gewebes verwendet. Eine herkömmliche Mikrodialysesonde ist beispielsweise durch einen Sondenkörper und eine von diesem hervorstehende Sondennadel gegeben. Beim Einführen in ein Gewebe, wie etwa menschliches oder tierisches Körpergewebe, wird die Sondennadel vollständig von dem Gewebe aufgenommen und der Sondenkörper verbleibt auf der Oberfläche des Gewebes, In einer Sondennadel sind z. B. konzentrisch verlaufende Zu- und Ableitungen für eine Perfusionslösung vorgesehen, wobei ein Bereich am Ende der Zuleitung eine Dialysemembran aufweist. Bei der Dialyse kommt es an der Dialysemembran zum Konzentrationsausgleich zwischen der Perfusionslösung und dem Umgebungsmilieu des Gewebes, in das die Sondennadel eingebracht wurde, wobei permeable gelöste Stoffe ausgetauscht werden. Nach dem Konzentrationsausgleich wird die Perfusionslösung über die Ableitung zurückgeführt.

Eine Bauform einer Mikrodialysesonde ist aus der DE 199 37 099 A1 bekannt, bei dieser Sonde werden sowohl die Zuleitung als auch die Ableitung in Form von getrennten Hohlkanälen nebeneinander vorgesehen. Hierfür werden beispielsweise zwei Röhren nebeneinander angeordnet, die durch ihren Zuschnitt eine gemeinsame Spitze zum Einführen in ein Gewebe bilden, und die in einem Bereich, in dem sie aneinander grenzen, jeweils eine Öffnung aufweisen, um die Umleitung der Perfusionslösung von einer Röhre in die andere Röhre zu ermöglichen. Innerhalb einer Röhre ist eine Dialysehohlfaser angeordnet und in dem Bereich der Hohlfaser weist die Röhre abschnittsweise Aussparungen auf, um die Hohlfaser mit dem Umgebungsmilieu in Kontakt zu bringen. In einer anderen Ausfiihrungsform wird eine sternförmige Stützstruktur in eine Dialysehohlfaser eingebracht. An den Spitzen der Stützstruktur liegt die Innenfläche der Hohlfaser auf, so dass sich zwischen der Stützstruktur und der Hohlfaser Durchgangskanäle bilden. In einem Bereich der Spitze der Sondennadel ist die Stützstruktur derart unterbrochen, dass die Hohlkanäle miteinander verbunden sind. Einer der Hohlkanäle dient als Zuleitung für die Perfusionslösung, die in dem Übergang der Kanäle in die anderen Kanäle übergeführt wird, welche als Ableitung dienen.

Bei diesen Mikrodialysesonden nach dem Stand der Technik ist entweder eine umfangreiche Stützstruktur wie ein Röhrensystem gegeben, wodurch eine Dialysemembran nicht unnötig zum Beispiel durch Druck durch das Gewebe oder beim Eindringen in das Gewebe beansprucht wird. Die Außenfläche der Stützstruktur ist dabei jedoch im Verhältnis wesentlich größer als ein zur Dialyse freigelassener Bereich. Um eine ausreichende Dialysefläche zu erreichen, müssen die Sondennadeln daher entsprechend lang und dick gebaut werden. Bei einer Stützstruktur im Inneren einer Dialysehohlfaser wird zwar eine Dialysefläche wesentlich vergrößert, jedoch erhält die Dialysemembran keinen ausreichenden Schutz gegenüber äußeren Einflüssen. '

Das Dokument WO 00/10464 offenbart eine Dialysesonde, beider eine Hohlfasermembian um einen stabförmigen Membranträger herumgebogen wird.

Bei der Herstellung derartiger Mikrodialysesonden sind eine Vielzahl von Einzelteilen in-oder aneinander zu setzen, so dass eine große Zahl an Übergangsbereichen oder Stoßkanten zwischen den einzelnen Teilen, insbesondere zwischen einer Dialysemembran und einer Stützstruktur entstehen. Da ein Dialysemembranmaterial beim Einbringen in ein Gewebe, das heißt wenn es feucht wird, aufquillt und sich ausdehnt, stellen Übergangsbereiche zwischen dem Membranmaterial und der Stützstruktur Schwachstellen einher Mikrodialysesonde dar. Im Allgemeinen ist ein Dialysemembranmaterial zwar flexibel, jedoch verbleibt es nicht von selbst in einer gebogenen oder gekrümmten Form, deshalb müssen ausreichend Stütz- und Befestigungsvorkehrungen getroffen werden, um eine Mikrodialysemembran in einer gewünschten Form zu halten. Diese Stützstrukturen schränken jedoch meist den freien Dialysebereich stark ein.

Es ist die Aufgabe der vorliegenden Erfindung ein Verfahren zur Herstellung einer Mikrodialysesonde aufzuzeigen, das eine Dialysemembran auf einfache Weise in eine gewünschte Form, bzw. einen gewünschten Verlauf, bringt und in dieser Form fixiert, wobei nur wenige Einzelteile verwendet werden.

Diese Aufgabe wird nach der Erfindung durch ein Verfahren zur Herstellung einer Mikrodialysesonde nach dem Anspruch 1 gelöst. Vorteilhafte Ausführungsformen gehen aus den Unteransprüchen hervor.

Eine Mikrodialysesonde weist einen Sondenkörper auf, aus dem eine Sondermadel hervorsteht, die in ein Gewebe eingeführt werden kann. Weiter weist die Mikrodialysesonde eine Zuleitung und eine Ableitung für eine Perfusionslösung und eine Dialysemembran auf, wobei die Membran wenigstens einen Teil der Zu- und/oder Ableitung bilden kann. Die Sondennadel wird durch einen langgestreckten Rahmen gebildet. Der Rahmen weist in einer oder mehreren seiner Umfangsoberflächen wenigstens eine Aussparung auf, die zu einer Außenseite des Rahmens hin offen ist und zur Ausnahme der Zuleitung und/oder der Ableitung einer Perfusionsflüssigkeit dient.

Vorzugsweise weist der Rahmen einen rechteckförmigen Querschnitt mit einer im Vergleich zur Breite geringeren Höhe auf. Die Höhe und die Breite sollten so gering wie möglich gewählt werden, da der Rahmen zum Einführen in ein menschliches Gewebe verwendet wird und dadurch die Unannehmlichkeit für einen Anwender gering gehalten wird. Natürlich kann der Rahmen auch einen beliebigen anderen Querschnitt aufweisen. An einem von dem Sondenkörper entfernt gelegenen distalen Endbereich ist am dem Rahmen eine Spitze zum Einführen in das Gewebe ausgebildet. Vorzugsweise wird die Spitze durch ein Zusammenlaufen der schmalen sich gegenüberliegenden Seiten des Rahmens in Richtung der Mitte des Rahmens ausgebildet, wie es z. B. in der Patentanmeldung mit dem Titel "Nadelspitze" der Anmelderin beschrieben ist, die auf die Anmelderin zurückgeht und den gleichen Anmeldetag wie die vorliegende Anmeldung aufweist. Vorzugsweise ist der Rahmen als ein einteiliges Formstück ausgebildet. Dabei kann der Rahmen einen Teil des Sondenkörpers bilden, indem er zum Beispiel an seinem der Spitze gegenüberliegenden Ende derart verbreitert ausgeführt ist, dass die Verbreiterung eine Gehäuseseite des Sondenkörpers bildet. Bevorzugt weist der Rahmen wenigstens teilweise eine Flexibilität auf, durch die er grundsätzlich in einem gewissen Maß biegsam ist, jedoch nicht in einer gebogenen Stellung verbleibt. Durch diese Flexibilität kann die Sondennadel kleinen Bewegungen oder Drücken des Gewebes nachgeben, wodurch das Tragen der Mikrodialysesonde für einen Anwender angenehmer wird. Bevorzugt ist der Rahmen aus Kunststoff gefertigt, das diese Flexibilität ermöglicht. Der Rahmen kann jedoch ebenso aus Metall gefertigt werden. Ferner kann an einem Kunststoffrahmen eine Metallspitze angebracht werden, um das Einführen in ein Gewebe zu erleichtern.

An wenigstens einer Umfangfläche des Rahmens, d. h. beispielsweise' an einer der in Längsrichtung eines rechteckförmigen Rahmens verlaufenden Seitenflächen, vorzugsweise an der breiteren Seitenfläche, sind zur Aufnahme der Zuleitung und vorzugsweise auch der Ableitung offene Aussparungen vorgesehen, die im Wesentlichen in der Längsrichtung des langgestreckten Rahmens verlaufen. Demnach ist eine Aussparung in den Rahmen integriert, das heißt sie ist in den Rahmen eingelassen. Die Zuleitung und die Ableitung gehen vorzugsweise an einer Umkehrung ineinander über, die innerhalb einer Aussparung liegt. Die Aussparungen werden beispielsweise durch Ätztechnik in den Rahmen eingebracht, es können jedoch auch andere Techniken wie Fräsen oder Schneiden verwendet werden. Bei einem Rahmen, der auch einen Teil des Sondenkörpers bildet, beginnt eine Aussparung vorzugsweise bereits in dem den Sondenkörper bildenden Teil. Weiter erstreckt sich eine Aussparung vorteilhaft bis zur Spitze des Rahmens, das heißt bis zum distalen Endbereich der Sondennadel. Dadurch kann durch die Aussparung in dem Rahmen zwischen dem Rahmen und dem Sondenkörper eine Öffnung ausgebildet werden, die innerhalb der Querschnittsfläche des Rahmens liegt.

Die Umkehrung zwischen der Zu- und der Ableitung kann durch den Verlauf der Aussparung gebildet. werden Hierfur kann in dem Rahmen wenigstens eine Aussparung derart ausgebildet sein, dass eine erste entlang dem Rahmen verlaufende Vertiefung die Zuführung bildet und eine zweite Vertiefung, die neben der ersten Vertiefung angeordnet ist, die Ableitung bildet. Die erste und die zweite Vertiefung gehen in einem distalen Endbereich des Rahmens huber und bilden dadurch die Umkehrung. Die erste Vertiefung, die zweite Vertiefung und die Umkehrung bilden gemeinsam die Aussparung. Der Querschnitt der Vertiefungen in dem Rahmen ist vorzugsweise an seinen im Inneren des Rahmens liegenden Ecken abgerundet. Durchläuft eine Flüssigkeit diese Vertiefungen, ergibt sich aus den abgerundeten Ecken ein günstiges Strömungsverhalten der Flüssigkeit Der Querschnitt der Vertiefungen kann jedoch eine beliebige andere Form aufweisen, solange er nach oben aus der Umfangsfläche des Rahmens offen ist. Die Umkehrung verläuft vorzugsweise in einem kreisförmigen Bogen um 180° von der ersten Vertiefung zur zweiten Vertiefung. Durch einen runden Verlauf der Umkehrung wird ein günstiges Strömungsverhalten einer Flüssigkeit, die durch die Aussparung läuft, erhalten. Die erste und zweite Vertiefung sind vorzugsweise an der gleichen Umfangsfläche des Rahmens angeordnet. Falls der Rahmen mehrere in Längsrichtung des Rahmens verlaufende Umfangsflächen aufweist, wie etwa ein rechteckförmiger Rahmen, können die Vertiefungen jedoch auch an verschiedenen Seiten, insbesondere an gegenüberliegenden Seiten vorgesehen sein. In diesem Fall kann die Umkehrung zwischen der Zuleitung und der Ableitung durch eine Bohrung durch den Rahmen vorgesehen sein, der die erste und zweite Vertiefung verbindet.

Über einer Aussparung wird wenigstens eine Dialysemembran in Form eines Membranblattes, einer Membranschicht oder einer Membranlage auf der Umfangsfläche des Rahmens angebracht. Die Membranschicht wird an dem gesamten Oberflächenrand um die Aussparung herum mit dem Rahmen verbunden, zum Beispiel durch Laminieren oder Verkleben. Dadurch bildet sich unterhalb der Dialysemembran in dem Bereich zwischen der Aussparung und der Membran ein Hohlkanal für die Zu- und Ableitung.

Vorzugsweise wird eine einzige Membranschicht verwendet, die bereits in einem Rahmenbereich innerhalb des Sondenkörpers beginnt, sich über die gesamte Aussparung erstreckt und an dem distalen Endbereich des Rahmens endet. Sind jedoch Aussparungen, bzw. Vertiefungen, an verschiedenen Seiten des Rahmens vorgesehen, ist es vorteilhaft für jede Seite eine getrennte Membranschicht zu verwenden.

Der Rahmen Kann wenigstens eine Aussparung aufweiser, die den Rahmen zumindest teilweise durchtrennt, so dass sich innerhalb des Rahmens ein in Längsrichtung verlaufender Schlitz bildet. Vorzugsweise wird der Schlitz durch Ätzen in einen Rahmen, der zum Beispiel aus Kunststoff besteht, eingebracht. Dabei ist es vorteilhaft, die im Inneren des Schlitzes liegenden Seitenwände des Schlitzes konkav auszubilden. Die Wände können jedoch eine beliebige andere Form aufweisen. Vorzugsweise beginnt der Schlitz in einem Rahmenbereich innerhalb des Sondenkörpers, wodurch innerhalb des Rahmenquerschnitts eine Öffnung am Sondenkörper ausgebildet werden kann. Der Schlitz endet vorzugsweise kurz vor dem distalen Endbereich des Rahmens mit einem abgerundeten Bereich.

In dem Schlitz ist eine Dialysemembran in Form einer Hohlfaser derart angeordnet, dass sie in einem Umkehrbereich umgelenkt wird und dadurch zwei Strecken der Hohlfaser annähernd nebeneinander zu liegen kommen. Vorzugsweise ist der Umkehrbereich in der Rundung des Schlitzes an dem distalen Endbereich des Rahmens vorgesehen. Die Hohlfasermembran wird in diesem Umkehrbereich um 180° gebogen, so dass zwei Hohlfaserstrecken nebeneinander liegen, wobei eine die Zuleitung und eine die Ableitung für eine Perfusionslösung bildet. Die Dialysemembranhohlfaser wird in dem Umkehrbereich befestigt, beispielsweise durch ein Haftmittel oder auch durch einen Stift um den sie gelegt wird, der mit dem Rahmen verbunden ist.

Vorzugsweise weist die Höhe des Querschnitts des Rahmens die gleiche Größe auf wie der Außendurchmesser der Hohlfasermembran. Der Schlitz ist vorteilhafterweise genauso breit wie zweimal der Außendurchmesser der Hohlfasermembran. Dadurch füllen die beiden nebeneinander verlaufenden Hohlfasermembranen den durch den Schlitz in dem Rahmen entstehenden Zwischenraum genau aus, ohne dass die Hohlfasermembran verformt wird oder über die Rahmenoberfläche hinausragt. Beginnt der Schlitz bereits in einem Rahmenbereich innerhalb des Sondenkörpers, ist es möglich, dass eine einteilige Hohlfasermembran aus dem Sondenkörper austritt, entlang des Schlitzes bis zu dem Umlenkbereich und wieder zurück in den Sondenkörper verläuft. Dadurch sind keinerlei Verbindungsstellen innerhalb der Sondennadel erforderlich, sondern die Dialysemembranoberfläche wird innerhalb des Gewebes von einer einzigen durchgehenden Oberfläche gebildet. Vorzugsweise liegt die Hohlfasermembran zwischen dem Umkehrbereich und ihrem Anfangs-, bzw. Endbereich frei, das heißt es sind in der Sondennadel neben der Befestigung in dem Umlenkbereich keinerlei weitere Befestigungspunkte für die Hohlfasermembran notwendig. Besonders bevorzugt tritt die Hohlfasermembran aus dem Sondenkörper durch eine Öffnung, die von dem Rahmen und dem Sondenkörper durch den Schlitz ermöglicht wurde, aus dem Sondenkörper aus und tritt nach der Umlenkung wieder durch eine solche Öffnung in den Sondenkörper ein. In einer Ebene der Umfangsfläche des Rahmens können über den Schlitz mehrere flache Vorsprünge hinausragen, die über die eingespannte Hohlfasermembran ragen und der Führung der in diesem Teil freiliegenden Hohlfaser dienen.

Es ist möglich zwischen der Hohlfaserstrecke für die Zuleitung und der Hohlfaserstrecke für die Ableitung eine Trennfläche vorzusehen. Durch eine derartige Trennfläche könnte ein kontinuierlicher Konzentrationsausgleich auf dem Hinweg in der Zuleitung und dem Rückweg in der Ableitung einer Perfusionslösung gewährleistet werden ohne, dass eine Wechselwirkung zwischen der Zuleitung und der Ableitung aufgrund des Konzentrationsunterschiedes in diesen Leitungen entsteht.

Um derartige Querdiffusionen zwischen der Zu- und der Ableitung zu vermeiden ist es auch möglich, die Hohlfaserdialysemembran entlang einer Teilstrecke der Zu- oder Ableitung mit einer nichtpermeablen Schicht zu umgeben, so dass in diesem Bereich keine Diffusion stattfindet. Hierfür wird die Membran vor dem Einbringen in dem Rahmen z. B. mit Kunststoff, vorzugsweise einem biokompatiblen Klebstoff, über eine Teilstrecke verschlossen. Die verschlossene Teilstrecke beträgt vorteilhafterweise ca. 20 bis 50 % der Gesamtholfaserstrecke für eine Zu- oder Ableitung. Um Querdiffusionen möglichst effizient zu unterbinden, beginnt eine verschlossene Teilstrecke gleich nach dem Austreten der Dialysehohlfassrmembran aus dem Sondenkörper, so dass der verschlossene Teil am Sondenkörper angeordnet ist. Der verbleibende Teil in Richtung der Nadelspitze bleibt zur Dialyse offen. Eine solche Anordnung der verschlossenen Hohlfaserstrecke lässt die Zuund Ableitung in dem distalen Endbereich der Dialysenadel frei, der am weitesten in das Gewebe eingeführt wird und um den der größte Flüssigkeitsaustausch stattfindet. Weiter wird die verschlossene Hohlfaserteilstrecke bevorzugt an der Zuleitung vorgesehen und die Ableitung über die gesamte Strecke von der Nadelspitze bis zum Sondenkörper unbehandelt belassen. Vorzugsweise wird eine farbige Schicht verwendet, so dass die Positionierung der Membran innerhalb des Rahmens an einer gewünschten Stelle erleichtert wird.

Eine Dialyseoberfläche, die von der Dialysemembran gebildet wird, erstreckt sich die gesamte Länge der Aussparung, die vorzugsweise bereits innerhalb des Sondenkörpers beginnt. Daher erstreckt sich die Dialyseoberfläche über die gesamte Länge der Sondennadel. Die Länge der Sondennadel muss also nicht länger sein als die Strecke, die für eine ausreichende Dialyse notwendig ist. Ferner wird insbesondere bei der Ausführungsform mit einem Schlitz und einer Hohlfasermembran die Dialyseoberfläche maximiert, da kaum Stützvorkehrungen oder Befestigungsvorrichtungen die Dialysefläche beeinträchtigen. Es ergibt sich daher ein für die Dialyse optimales Verhältnis von Membranoberfläche zu Leitungsvolumen, d. h. die durchgeführte Perfusionslösung kann über eine maximierte Fläche mit dem Gewebe in Kontakt treten, es entstehen keinerlei Toträume innerhalb der Leitungen. Der Dialysevorgang ist bei dem genannten Ausführungsbeispielen sowohl in der Zu- als auch in der Ableitung möglich, das heißt zweimal über die gesamte Nadellänge. Dadurch kann die Gesamtnadellänge, das heißt die Länge des Rahmens, weiter verkürzt werden. Bei einer Mikrodialysemembran wird auch die Zahl der übergänge zwischen einer Dialysemembran und weiteren Nadelbauteilen reduziert, insbesondere bei einem Rahmen mit Schlitz und einer Hohfasermembran gibt es keine solchen Übergänge mehr. Für einen Anwender erhöht sich der Tragekomfort bei Verwendung einer erfindungsgemäßen Mikrodialysesonde, da diese besonders schmal und kurz ausgebildet werden kann, außerdem wird das Tragen der Sonde durch die Flexibilität der Nadel angenehmer.

Grundsätzlich wäre es auch denkbar eine Aussparung nur teilweise nach außen offen zu gestalten und einen anderen Teil z. B. als Mittenbohrung durch den Kanal auszubilden. Ferner ist es denkbar zum Beispiel eine Zuleitung und mehrere Ableitungen vorzusehen. Hierfür könnten zum Beispiel drei Vertiefungen in einem Rahmen vorgesehen sein, wobei eine Vertiefung durch eine Umkehrung in die beiden anderen Vertiefungen übergeht. Dabei könnte zum Beispiel ein Rahmen mit einem dreieck- oder sechseckförmigen Querschnitt verwendet werden, der eine Vertiefung an je einer Seite aufweist, wobei eine Vertiefung durch je eine Bohrung mit den beiden anderen Vertiefungen verbunden ist. Grundsätzlich kann auch bei einer Ausführungsform mit einer Aussparung in Form einer Vertiefung anstelle des Aufbringens einer Membranschicht über der Vertiefung, eine Hohlfasermembran innerhalb der Vertiefung verlegt werden.

Vorzugsweise ist der Rahmen derart flexibel, dass er noch ausreichende Steifigkeit aufweist, um als selbststechende Sondennadel verwendet zu werden. Natürlich ist es aber auch möglich, den Rahmen durch eine Einführhilfe in dem Gewebe zu platzieren und nach dem Einführen die Einfuhrhilfe zu entfernen.

Eine Mikrodialysesonde ist neben einer Konzentrationsmessung auch für eine Viskositätsmessung innerhalb eines Gewebes geeignet

Die Aufgabe der Erfindung wird durch ein Verfahren zur Herstellung einer Mikrodialysesonde erfüllt, bei dem eine Dialysemembran in eine vorbestimmte zumindest teilweise gebogene Form gebracht wird und in oder an einem Rahmen, der eine Sondennadel bildet, angeordnet ist Hierfür liegt zunächst die Dialysemembran an einem Formgebungsmittel an und wird dann durch Biegen, bzw. Formen des Formgebungsmittels in eine vorbestimmte Form gebracht. Anschließend wird ein Haftmittel, bzw. ein Verbindungsmittel, wie etwa ein Kitt oder ein Kleber, zumindest teilweise an einer Biegestelle der Dialysemembran angebracht, so dass die Membran in der vorbestimmten Form gehalten wird. Nach dem Anbringen des Haftmittels, das eine versteifende Wirkung hat, wird das Formgebungsmittel von der Dialysemembran entfernt.

Bei einem bevorzugten Verfahren liegt die Dialysemembran in Form einer Hohlfaser vor und wird von einem Filament, bzw, einem Faden oder einem Draht, als Formgebungsmittel durchzogen. Dabei ist es vorteilhaft, wenn das Filament einen Außendurchmesser aufweist, der annähernd dem Innendurchmesser der Membranhohlfaser entspricht, jedoch ausreichend Abstand frei lässt, um das Filament entfernen zu können, sobald die Membranhohlfaser in ihrer endgültigen Form befestigt ist.

Vorzugsweise wird ein vorgefertigter Rahmen, oder eine vorgefertigte Stützstruktur verwendet, durch dessen Ausgestaltung die Formgebung der Dialysemembran, bzw. der Membranhohlfaser, bestimmt wird. Die Dialysemembran kann dann mit Hilfe des Formgebungsmittels in diese vorgefertigte Form eingepasst werden und mit Hilfe des Haftmittels an ihren Biegestellen in dieser Form gehalten werden, wobei das Haftmittel die Dialysemembran zumindest teilweise mit dem Rahmen verbinden kann.

Bevorzugt wird als Filament ein Nylonfaden verwendet. Das Haftmittel kann zum Beispiel aus Cyanoacrylat, Silikon oder Epoxid bestehen. Es ist auch möglich als Haftmittel ein ein-oder zweiseitig druckempfindliches Klebeflächenmaterial zu verwenden. Das Haftmittel sollte Grundsätzlich auf das Material des Rahmens und der Dialysemembran abgestimmt werden, um eine schnelle und dennoch gut haftende Verbindung herstellen zu können.

Grundsätzlich ist es auch möglich, dass der Rahmen durch das Haftmittel, d. h. von dem Material des Haftmittels, gebildet wird. Hierbei kann die Dialysemembran durch das Formgebungsmittel in eine vorbestimmte Form gebracht werden und anschließend das Haftmittel in einer solchen Form zumindest teilweise an der Dialysemembran angebracht werden, dass die Form des Haftmittels zum Beispiel einer gewünschten Form für eine Dialysenadel entspricht. Hierfür ist es vorteilhaft, dass das Haftmittel nach dem Anbringen an der Dialysemembran eine versteifende Wirkung aufweist. Dabei ist darauf zu achten, dass eine ausreichende Fläche der Dialysemembran für die Dialyse frei bleibt. Es ist möglich das Haftmittel in einer Art Rohform um die Dialysemembran anzubringen und erst anschließend eine gewünschte Form herauszubilden, zum Beispiel durch Ätzen oder Schneiden.

Als Dialysemembran kann auch eine Membranschicht, bzw. ein Membranblatt verwendet werden, die zum Beispiel durch ein Blech als Formgebungsmittel in Form gebracht wird. Dadurch ist es möglich große Flächen einer Dialysemembran in eine gewünschte gebogene Form zu bringen, wie es beispielsweise beim Umwickeln von Rahmen oder Stützstrukturen erforderlich sein kann. Für das Verfahren der vorliegenden Erfindung ist es unerheblich, ob das Haftmittel zuerst an dem Rahmen oder der Dialysemembran angebracht wird. Bei der Auswahl der Befestigungspunkte sollte darauf geachtet werden, dass die Membran sich in feuchtem Zustand ausdehnt, so dass sie nach dem Befestigen keine Knicke oder Falten wirft, welche die gewünschte Form der Membran stören könnten.

Mit dem erfindungsgemäßen Verfahren kann zum Beispiel eine Mikrodialysesonde in einer oben beschriebenen Ausführungsform hergestellt werden. Wird beispielsweise für eine Sondennadel ein flacher langgestreckter Rahmen mit einer Aussparung in Form eines Schlitzes für die Zu- und die Ableitung einer Perfusionslösung verwendet, kann eine Hohlfasermembran mit Hilfe des erfindungsgemäßen Verfahrens entsprechend einer durch den Schlitz vorbestimmten Form innerhalb des Schlitzes angeordnet werden. Hierfür wird - die Hohlfasermembran mit Hilfe des sie durchlaufenden Filaments innerhalb des Schlitzes des Rahmens derart angeordnet, dass zwei Strecken der Hohlfaser nebeneinander zu liegen kommen und an einem abgerundeten Ende des Schlitzes vor einem distalen Endbereich des Rahmens eine Schleife um 180° bilden. Anschließend wird das Haftmittel zumindest an der Rundung des Schlitzes zwischen dem Rahmen und der Hohlfasermembran angebracht.. Nachdem die befestigende, bzw. versteifende Wirkung des Haftmittels eingetreten ist, kann das Filament aus der Membranhohlfaser entfernt werden. Dadurch ist die Membranhohlfaser innerhalb des Schlitzes verlegt und in diesen eingespannt. Die beiden Enden der Membranhohlfaser verlaufen vorzugsweise in einen Sondenkörper hinein. Eine derartige Sondennadel einer Mikrodialysesonde weist nach dem Einführen in ein Gewebe keinerlei Verbindungsstellen innerhalb des Gewebes auf.

Vor dem Einsetzen der Hohlfasermembran in den Rahmen, ist es vorteilhaft, eine Teilstrecke der Hohlfasermembran mit einer nichtpermeablen Schicht, wie etwa einem biokompatiblen Klebstoff, zu umgeben. Der Klebstoff verschließt über diese Teilstrecke die Dialysemembran, so dass entlang dieser Strecke kein Austausch stattfindet. Beim Einsetzen der Hohlfasermembran in den Rahmen wird diese verschlossene Teilstrecke vorzugsweise am Austritt der Zuleitung aus dem Sondenkörper eingeordnet. Zwischen dieser Zuleitung und einer daneben verlaufenden Hohlfaserstrecke für die Ableitung kann entlang der verschlossenen Teilstrecke in dem angrenzenden Bereich der Zu- und der Ableitung keine Wechselwirkung entstehen, so dass eine Querdiffusion von der Zu- zu der Ableitung unterbunden wird. Vorzugsweise wird eine farbige Schicht gewählt, um beim Einsetzen der Hohlfaserdialysemembran die Positionierung der verschlossenen Teilstrecke an einer gewünschten Stelle in dem Rahmen zu erleichtern.

Die Erfindung wird beispielhaft anhand der Zeichnung näher erläutert, wobei dargestellte Fort- und Weiterbildungen der Erfindung als zum Umfang der Erfindung gehörend betrachtet werden sollen. In der Zeichnung stellen dar:
Figur 1 eine perspektivische Ansicht und einen Querschnitt einer ersten Ausführungsform einer Mikrodialysesonde,
Figur 2 eine perspektivische Ansicht und einen Querschnitt nach einer zweiten Ausführungsform,
Figur 3 eine perspektivische Ansicht einer Mikrodialysesonde des Ausführungsbeispiels aus Figur 2 mit einem Sondenkörper,
Figuren 4a bis 4c eine dritte Ausführungsform der Erfindung und
Figuren 5a bis 5e eine Darstellung der Arbeitsschritte eines Verfahrens nach der' vorliegenden Erfindung.

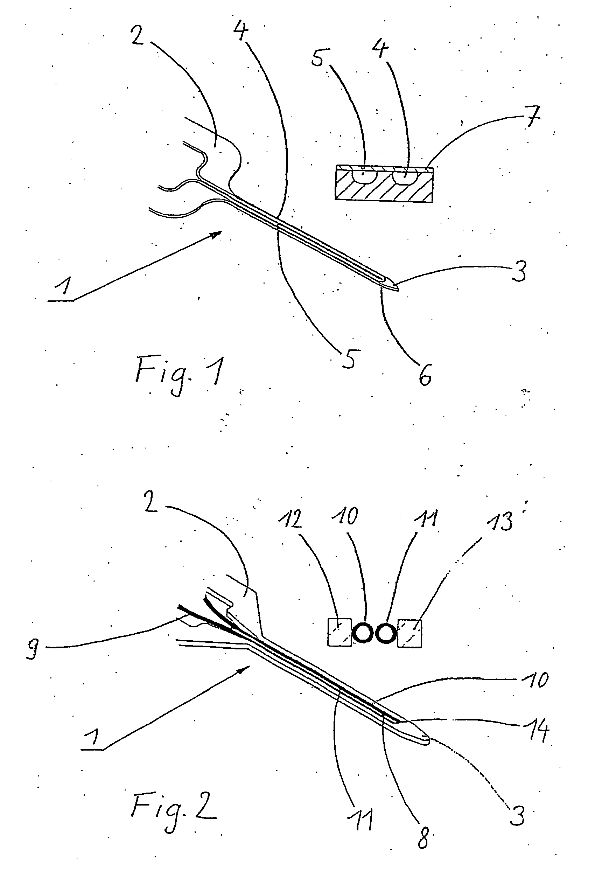

In Figur 1 ist eine Sondennadel einer Mikrodialysesonde dargestellt, die durch einen flachen langgestreckten Rahmen 1 gegeben ist. Der Rahmen 1 weist in einem hinteren Bereich eine Verbreiterung 2 als Teil eines Sondenkörpers auf. In einem distalen Endbereich ist an dem Rahmen 1 eine Spitze 3 zum Einführen in ein Gewebe ausgebildet. Der Querschnitt des Rahmens 1 ist rechteckförmig und weist zum Beispiel eine Höhe von 0,3 mm und eine Breite von 1,0 mm auf. Die Länge des Rahmens 1 ist auf die benötigte Dialysefläche abgestimmt und beträgt zum Beispiel ca. 10,0 mm. In einer Umfangsfläche des Rahmens 1 ist entlang des Rahmens 1 eine erste Vertiefung 4 und eine zweite Vertiefung 5 angeordnet, die parallel neben der ersten Vertiefung 4 verläuft. Wie in dem Querschnitt der Figur 1 zu sehen ist, sind die Kanten zwischen einem Boden und den Seitenwänden der Vertiefungen abgerundet. Vor dem distalen Endbereich des Rahmens 1 nahe der Spitze 3 gehen die erste Vertiefung 4 und die zweite Vertiefung 5 in einer Umkehrung 6 ineinander über. Die Umkehrung ist als halbkreisförmige Vertiefung ausgebildet, die sowohl in die ersten Vertiefung 4 als auch in die zweite Vertiefung 5 mündet, so dass diese miteinander verbunden sind. Die erste Vertiefung 4, die zweite Vertiefung 5 und die Umkehrung 6 bilden eine zu einer Außenseite des Rahmens 1 offene Aussparung gemäß der vorliegenden Erfindung zur Aufnahme einer Zuleitung und einer Ableitung für eine Perfusionslösung der Mikrodialysesonde. Dabei kann die erste Vertiefung 4 die Zuleitung und die zweite Vertiefung 5 die Ableitung bilden oder umgekehrt. Wie aus der Zeichnung ersichtlich ist, erstrecken sich die geraden Bereiche der Vertiefungen 4 und 5 bis in die Rahmenverbreiterung 2 hinein, so dass die Vertiefungen 4 und 5 in einem zusammengesetzten Zustand der Mikrodialysesonde, das heißt bei einer Verbindung des Sondenkörpers mit der Sondennadel, bis in den Sondenkörper hinein verlaufen. Auf der Umfangsfläche des Rahmens 1, in der die Vertiefungen 4 und 5 angeordnet sind, wird eine Dialysemembranschicht 7 angeordnet. Wie aus dem Querschnitt der Figur 1 zu entnehmen ist, verläuft die Membranschicht 7 über die gesamte Breite des Rahmens 1 und bedeckt die Vertiefungen 4 und 5. An den Auflageflächen der Membranschicht 7 auf der Oberfläche des Rahmens 1 wird die Schicht mit dem Rahmen verbunden, zum Beispiel durch Laminieren. Zwischen der Membranschicht und dem Rahmen 1 bildet sich ein Hohlkanal aus, durch den die Perfusionslösung geleitet werden kann. Nach dem Einführen der Dialysesonde in ein Gewebe, ist der gesamte Nadelbereich mit dem Gewebe umgeben. Beim Durchleiten einer Perfusionslösung durch den Hohlkanal kann ein Konzentrationsaustausch zwischen dem Umgebungsmilieu und der Perfusionslösung entlang der gesamten Länge des Rahmens 1 und über die gesamte Breite der Vertiefungen 4 und 5 erfolgen.

Als Material für die Membranschicht kann beispielsweise regenerierte Zellulose verwendet werden, wie etwa von Medicell oder Spektrum Labs. Der Rahmen 1 kann zum Beispiel aus Stahl, einem flüssigkristallinen Polymer oder Polybuthylenterephthalat (PBT) gebildet werden. Aber auch vergleichbare Metalle und Kunststoffe sind zur Herstellung des Rahmens geeignet. Vorteilhafterweise werden flexible Materialien verwendet, um der Sondennadel eine Biegsamkeit verleihen zu können, die das Tragen der Nadel für einen. Anwender angenehmer gestaltet.

In Figur 2 ist ein zweites Ausführungsbeispiel für einen langgestreckten Rahmen 1 einer Mikrodialysesonde gezeigt. Der Rahmen 1. ist in Längsrichtung von einem verbreiterten Bereich 2 bis zur Rahmenspitze 3 von einer Aussparung durchtrennt, die einen Schlitz 8 in dem Rahmen ausbildet, In der Verbreiterung 2 wird auch der Schlitz 8 verbreitert. Innerhalb des Schlitzes 8 wird eine Dialysemembran in Form einer Hohlfaser 9 derart angeordnet, dass zwei Hohlfaserstrecken 10 und 11 nebeneinander zu liegen kommen. Die Hohlfaserstrecken 10 und 11. bilden die Zu- bzw. Ableitung für die Perfusionslösung in die Sondennadel.

Wie aus dem Querschnitt in Figur 2 ersichtlich ist, wird durch den Schlitz 8 der Rahmen 1 abschnittsweise in zwei Schenkel 12 und 13 geteilt. Zwischen diesen Schenkeln 12 und 13 ist der Querschnitt der Hohlfaserstrecken 10 und 11 dargestellt. Der Zwischenraum Zwischen den Schenkeln 12 und 13 entspricht dabei zweimal der Größe des Außendurchmessers der Membranhohlfaser und die Höhe der Schenkel 12 und 13, bzw. des Rahmens 1, entspricht dem Außendurchmesser der Membranhohlfaser 9. Dabei ist darauf zu achten, dass sich die Membranhohlfaser in feuchtem Zustand ausdehnt und sich der Durchmesser dabei erweitert. Der Rahmen 1 ist auf die Ausmaße einer ausgedehnten bzw. gequollenen Membranhohlfaser abgestimmt.

Die Membranhohlfaser kann zum Beispiel mit Hilfe eines Verfahrens gemäß der vorliegenden Erfindung in den Schlitz 8 eingebracht werden. Dabei wird die Membranhohlfaser 9 an einer Umkehrung, bzw. Schleife 14 der Membran an einem abgerundeten Endbereich des Schlitzes vor dem distalen Endbereich des Rahmens befestigt Die Hohlfaserstrecken 10 und 11 können dann ohne weitere Befestigung zwischen den Schenkeln 12 und 13 zu liegen kommen. Beim Einführen der Sondennadel in ein Gewebe dringt der Rahmen 1 bis zur Verbreiterung 2 in das Gewebe ein. Dabei verläuft die Membranhohlfaser 9 durchgehend in das Gewebe hinein und aus diesem heraus. Es sind also keine Übergangsstellen zwischen dem Membranmaterial und dem Rahnenmaterial vorhanden. Wie in Figur 2 gezeigt ist, wird die Hohlfaser 9 von dem verbreiterten Schlitzbereich in der Verbreiterung 2 ausgehend in den Schlitz 8 des Rahmens 1 eingeführt. Innerhalb des Gewebes kommt das Umgebungsmilieu von zwei Seiten des Rahmens 1 mit der Dialysemembran in Kontakt, wodurch der Oberflächenbereich der Membranschicht, der an der Dialyse, bzw. dem Konzentrationsaustausch beteiligt ist, im Vergleich zur Ausführungsform nach Figur 1, weiter vergrößert werden. Ferner kann der Konzentrationsausgleich sowohl in der Zuleitung als auch in der Ableitung der Perfusionsflüssigkeit erfolgen.

Die Nadelspitze 3 wird sowohl in Figur 1 als auch in Figur 2 durch ein Aufeinanderzulaufen der schmalen Seiten des Rahmens 1 gebildet, wie es z. B. in der vorher angeführten Patentanmeldung näher ausgeführt ist.

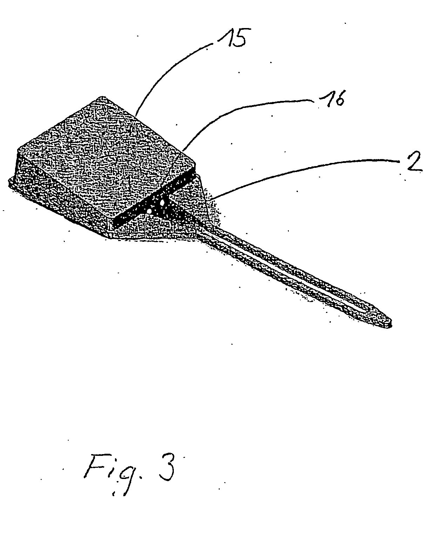

In Figur 3 ist eine Ausführungsform des Rahmens 1 aus Figur 2 gezeigt, auf dessen verbreiterten Bereich 2 ein Gehäuseteil 15 aufgesetzt ist. Die Verbreiterung 2 und das Gehäuseteil 15 bilden somit den Sondenkörper. Das Gehäuseteil 15 ist derart ausgebildet, dass es mit einer Unterseite auf den verbreiterten Bereich 2 des Rahmens 1 aufgesetzt werden kann. Dabei ragt ein Vorsprung 16 in die Verbreiterung des Schlitzes 8 hinein, der zwei runde Öffnungen aufweist, durch welche die Hohlfaserstrecken 10 und 11 geführt werden können. Auch an dem Übergang zwischen Membran und Sondenkörpers sind keinerlei Verbindungsstellen zwischen dem Membranmaterial und dem Sondenkörpermaterial erforderlich, sondern eine Perfusionslösung kommt ausschließlich mit dem Membranmaterial in Kontakt.

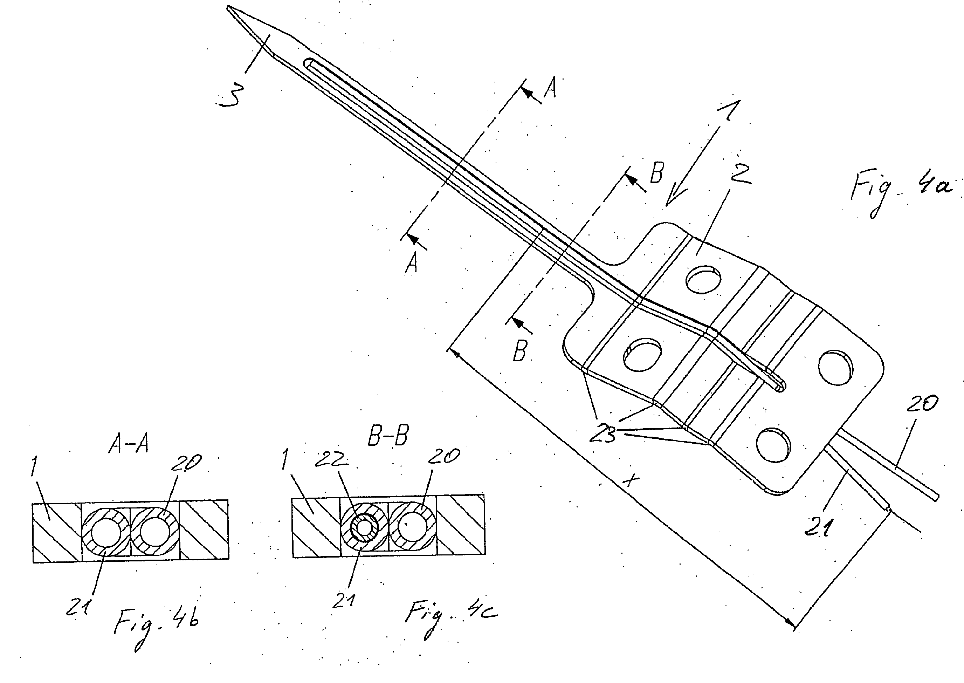

Die Figuren 4a bis 4c zeigen ein drittes Ausführungsbeispiel. Soweit nachfolgend nichts anderes beschrieben wird, entspricht das dritte Ausführungsbeispiel dem zweiten Ausführungsbeispiel.

Der als Dialysemembran dienende Hohlleiter ist einstückig gebildet und umfasst einen Zuleitungsabschnitt 20 und einen Ableitungsabschnitt 21, die bei der Rahmenspitze 3 ineinander übergehen, indem der Hohlleiter 20/21 dort eng gebogen ist. Der Hohlleiter 20/21 ist ein genau gearbeiteter Hohlfaden, vorzugsweise ein Nylonfaden, und weist die für die Dialysefunktion erforderliche Durchlässigkeit auf. Er bildet über seine gesamte Länge, insbesondere in seinem der Dialyse dienenden Abschnitt zwischen dem verbreiterten Bereich 2 des Rahmens 1 und der Rahmenspitze 3, einen genau definierten Hohlquerschnitt. Einer der beiden Abschnitte 20 und 21, im Ausführungsbeispiel der Abschnitt 21, weist einen größeren Strömungswiderstand auf als der sonst freie Strömungsquerschnitt des Hohlleiters 20/21. Um den größeren Strömungswiderstand zu erzielen, ist in dem Abschnitt 21 über die Länge X ein den Strömungsquerschnitt verengender Hohlleiter 22 integriert, der der flüssigen Substanz den erhöhten Strömungswiderstand entgegensetzt. Der Hohlleiter 22 oder allgemeiner ausgedrückt, der strömungserhöhende Einsatz, der nicht unbedingt als Hohlleiter gebildet sein muss, erstreckt sich im Abschnitt 21 über die gesamte Länge des verbreiterten Bereichs 2. Er reicht sowohl stromaufwärts als auch stromabwärts sogar ein Stück über den verbreiterten Bereich 2 hinaus. Zur Rahmenspitze 3 hin sollte er jedoch nur über solch eine Länge über den Bereich 2 hinausragen, innerhalb der ein Austausch mit der Körperflüssigkeit nicht stattfindet.

Der Hohlleiter 20/21 wird im dritten Ausführungsbeispiel auf besonders einfache Weise am Rahmen 1 befestigt. Für die Befestigung ist der verbreiterte Bereich 2 mehrfach quer zu dem Hohlleiter 20/21 hin und her geknickt, so dass er im Ergebnis faltenförmig ist. Die Knicklinien sind mit 23 bezeichnet. Die an den Knicklinien 23 gebildeten Abknickungen sind jedoch so flach, dass hierdurch der Strömungsquerschnitt im Hohlleiter 20/21 nicht beeinflusst wird.

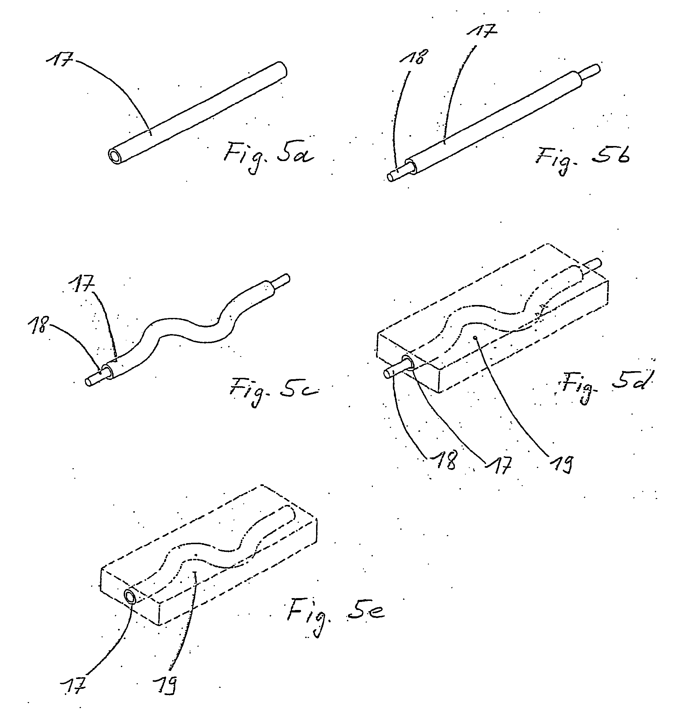

In den Figuren 5a bis 5e werden beispielhaft die verschiedenen Arbeitsschritte eines erfindungsgemäßen Verfahrens zur Herstellung einer Mikrodialysesonde veranschaulicht. In Figur 5a ist eine Dialysemembran in Form einer Hohlfaser 17 gezeigt, die zum Beispiel aus Zellulose bestehen kann. Derartige Membranen sind zwar flexibel und daher in verschiedene Formen biegbar, jedoch weisen herkömmliche Membranmaterialen eine Spannkraft auf, so dass die Membran in ihre ursprüngliche Form zurückkehrt, so bald die Kraft zum Verbiegen der Membran aufhört. Gemäß der vorliegenden Erfindung wird nun, wie in Figur 5b gezeigt ist, in die Membranhohlfaser ein Formgebungsmittel eingeführt. In dem gezeigten Beispiel ist das Formgebungsmittel ein Filament 18, dessen Außendurchmesser geringfügig kleiner ist als der Innendurchmesser der Membranhohlfaser. Als Filament kann beispielsweise ein Nylonfaden verwendet werden. Als nächstes wird, wie in Figur 5c gezeigt ist, die Membranhohlfaser durch Biegen des Filaments 18 in eine gewünschte Form gebracht, wie zum Beispiel in eine Form, die durch die Ausgestaltung einer Sondennadel oder eines Rahmens für eine Sondennadel vorgegeben ist. Zumindest an den Biegestellen liegt die Membranhohlfaser 17 an dem Filament 18 an. Zur Veranschaulichung des Herstellungsverfahrens wurde in der Figur 5c eine S-Form gewählt. Für einen praktischen Einsatz des erfindungsgemäßen Verfahrens zum Beispiel bei einem in Figur 2 gezeigten Rahmen einer Sondennadel wird die Membranhohlfaser lediglich, einmal um 180° umgebogen so dass zwei Membranhohlfaserstrecken nebeneinander zu liegen kommen.

Das Filament ist bei dem erfindungsgemäßen Verfahren lediglich als Hilfsmittel zur Führung der Membranhohlfaser vorgesehen, die entlang einer vorgegebenen Form die zum Beispiel durch die Ausgestaltung einer Stützstruktur oder eines Rahmens vorgegeben ist, verlegt werden soll. Es ist aber auch möglich, dass das Filament in verschiedene Formen verbiegbar ist und diese Form nach Beendigung der Krafteinwirkung beibehält, wie es beispielsweise bei einem Draht der Fall ist. Mit einem derartigen Filament kann die Membranhohlfaser auch ohne eine Stützstruktur oder einen Rahmen in eine gewünschte Form gebracht werden.

Nachdem die Hohlfasermembran in eine gewünschte Form gebracht wurde, wird ein Haftmittel wenigstens an einer Biegestelle der Membranhohlfaser angebracht. In Figur 4d ist die Hohlfasermembran 17 mit dem Filament 18 und mit einem Haftmittel 19 gezeigt, das vollständig um die Membranhohlfaser angebracht wurde. Für die vorliegende Erfindung ist es jedoch ausreichend, wenn das Haftmittel in Bereichen einer Biegestelle derart angebracht wird, dass ein Zurückbiegen der Membran nicht mehr möglich ist. Wenigstens die gerade verlaufenden Membranhohlfaserstrecken brauchen nicht mit dem Haftmittel fixiert zu werden. Nach dem Anbringen des Haftmittels 19 wird das Filament 18 aus der Dialysemembran entfernt. Um diesen Verfahrensschritt problemlos durchführen zu können, ist darauf zu achten, dass ein Filament, das seine Biegeform beibehält, wie etwa ein Draht, derart leicht biegbar ist, dass es einfach aus der geformten Membranhohlfaser herausgezogen werden kann, ohne dass dabei die Membran beschädigt wird oder eine Haftverbindung gelöst wird. Das in Figur 5e gezeigte Formteil, das aus der Membranhohlfaser 17 und dem Haftmittel 19 besteht, kann anschließend durch weitere Verarbeitungsschritte, wie etwa Ätzen, Schneiden oder Fräsen, weiterbearbeitet werden, so dass auch das Formteil eine gewünschte Form annimmt.

Grundsätzlich kann, wie oben beschrieben, auch eine Dialysemembranschicht, ein Dialysemembranblatt, oder dergleichen mit dem erfindungsgemäßen Verfahren geformt werden. Als Formgebungsmittel kann dabei zum Beispiel ein großflächiges Blech, ein Gitter oder mehrere über die Dialysemembranschicht verteilte Drähte oder dergleichen verwendet werden.

Als Haftmittel kann zum Beispiel Cynoacrylat, Silikon oder Epoxid verwendet werden, aber auch druckempfindliche Klebeflächenmaterialien, beispielsweise in Streifenform. Ferner können Haftmittel verwendet werden, die zum Beispiel durch UV-Licht ausgehärtet werden können.

Grundsätzlich ist es auch möglich, das Verfahren für Membranen anzuwenden, die in anderen Dialyseeinrichtungen als einer Mikrodialysesonde verwendet werden. Dialyseeinrichtungen sind zum Beispiel auch bei verschiedenen chemischen oder biologischen Untersuchungsverfahren zum Beispiel zur Messung der Viskosität oder der Konzentration von Bedeutung.

Die Erfindung wurde beispielhaft anhand der dargestellten Ausführungsformen beschrieben.

### Bezugszeichen

- 1: Rahmen
- 2: Verbreiterung
- 3: Rahmenspitze
- 4: erste Vertiefung
- 5: zweite Vertiefung
- 6: Umkehrung
- 7: Dialysemembranschicht
- 8: Schlitz
- 9: Membranhohlfaser
- 10: Hohlfaserstrecke
- 11: Hohlfaserstrecke
- 12: Schenkel
- 13: Schenkel
- 14: Umkehrung
- 15: Sondengehäuseteil
- 16: Vorsprung
- 17: Membranhohlfaser
- 18: Filament
- 19: Haftmittel

## Patentansprüche

1. Verfahren zur Herstellung einer Mikrodialysesonde, bei dem eine Dialysemembran (17) in eine vorbestimmte zumindest teilweise gebogene Form in und/oder an einem Rahmen gebracht wird, der die Sondennadel bildet, wobei
a) die Dialysemembran (17) an einem Formgebungsmittel (18) wenigstens teilweise anliegt,
b) die Dialysemembran (17) durch Biegen des Formgebungsmittels (18) in die vorbestimmte Form gebracht wird,
c) durch Anbringen eines Haftmittels (19) zumindest teilweise an einer Biegestelle der Dialysemembran (17) die Membran (17) in der vorbestimmten Form gehalten wird, wobei das Haftmittel (19) nach dem Anbringen an der Dialysemembran (17) eine versteifende Wirkung hat, und
d) nach dem Anbringen des Haftmittels (19) das Formgebungsmittel (18) von der Dialysemembran (17) entfernt wird.

2. Verfahren nach Anspruch 1, **dadurch gekennzeichnet, dass** die Dialysemembran in Form einer Hohlfaser (17) ausgebildet ist und von einem Filament (18) als Formgebungsmittel durchzogen wird.

3. Verfahren nach Anspruch 1 oder 2, **dadurch gekennzeichnet, dass** der Rahmen vorgefertigt ist und durch seine Ausgestaltung die Formgebung der Dialysemembran (17) bestimmt.

4. Verfahren nach dem vorhergehenden Anspruch, **dadurch gekennzeichnet, dass** das Haftmittel (19) die Dialysemembran (17) zumindest teilweise mit dem Rahmen verbindet.

5. Verfahren nach einem der Ansprüche 1 oder 2, **dadurch gekennzeichnet, dass** das Haftmittel (19) den Rahmen bildet.

6. Verfahren nach einem der vorhergehenden Ansprüche, **dadurch gekennzeichnet, dass** das Filament (18) ein Faden ist, vorzugsweise ein Nylonfaden.

7. Verfahren nach einem der vorhergehenden Ansprüche, **dadurch gekennzeichnet, dass** das Haftmittel (19) Cyanoacrylat, Silikon und/oder Epoxid umfasst.

8. Verfahren nach einem der vorhergehenden Ansprüche, **dadurch gekennzeichnet, dass** das Haftmittel (19) ein ein- oder zweiseitiges druckempfindliches Klebeflächenmaterial ist.

9. Verfahren nach einem der vorhergehenden Ansprüche, **dadurch gekennzeichnet, dass** vor dem Einbringen der Hohlfaserdialysemembran (17) eine Teilstrecke der Hohlfaserdialysemembran (17) mit einer nichtpermeablen Schicht umgeben wird.

## Claims

1. Method for the production of a microdialy-sis probe, in which a dialysis membrane (17) is brought into a predetermined, at least partially bent shape in and/or at a frame that forms the probe needle, wherein
a) the dialysis membrane (17) rests at least partially against a shaping means (18),
b) the dialysis membrane (17) is brought into the predetermined shape by bending the shaping means (18),
c) the membrane (17) is maintained in the predetermined shape by applying an adhesive agent (19) at least partially at a bend point of the dialysis membrane (17), wherein the adhesive agent (19) has a stiffening effect after the application to the dialysis membrane (17), and
d) the shaping means (18) is removed from the dialysis membrane (17) after the application of the adhesive agent (19).

2. Method of claim 1, **characterized in that** the dialysis membrane is designed in the form of a hollow fibre (17) and is drawn through by a filament (18) as the shaping means.

3. Method of claim 1 or 2, **characterized in that** the frame is prefabricated and determines by its configuration the shaping of the dialysis membrane (17).

4. Method of the preceding claim, **characterized in that** the adhesive agent (19) connects the dialysis membrane (17) at least partially to the frame.

5. Method of claim 1 or 2, **characterized in that** the adhesive agent (19) forms the frame.

6. Method of one of the preceding claims, **characterized in that** the filament (18) is a thread, preferably a nylon thread.

7. Method of one of the preceding claims, **characterized in that** the adhesive agent (19) comprises cyanoacrylate, silicone and/or epoxide.

8. Method of one of the preceding claims, **characterized in that** the adhesive agent (19) is a one-sided or two-sided pressure-sensitive adhesive tape material.

9. Method of one of the preceding claims, **characterized in that** before the introduction of the hollow fibre dialysis membrane (17) a section of the hollow fibre dialysis membrane (17) is surrounded with a non-permeable layer.

## Revendications

1. Procédé pour la fabrication d'une sonde de microdialyse, dans lequel une membrane de dialyse (17) est amenée, sous une forme prédéterminée au moins partiellement cintrée, dans et/ou sur un cadre qui forme l'aiguille de sonde, dans lequel
a) la membrane de dialyse (17) est appliquée au moins partiellement contre un moyen de mise en forme (18),
b) la membrane de dialyse (17) est amenée à la forme prédéterminée par cintrage du moyen de mise en forme (18),
c) par application d'un moyen adhésif (19) au moins partiellement sur un emplacement de cintrage de la membrane de dialyse (17), la membrane (17) est maintenue dans la forme prédéterminée, et le moyen adhésif (19) a un effet de rigidification après son application contre la membrane de dialyse (17), et
d) après application du moyen adhésif (19), le moyen de mise en forme (18) est enlevé de la membrane de dialyse (17).

2. Procédé selon la revendication 1, **caractérisé en ce que** la membrane de dialyse est réalisée sous la forme d'une fibre creuse (17) et est traversée par un filament (18) en guise de moyen de mise en forme.

3. Procédé selon la revendication 1 ou 2, **caractérisé en ce que** le cadre est préfabriqué, et, de par sa conception, il détermine la conformation de la membrane de dialyse (17).

4. Procédé selon la revendication précédente, **caractérisé en ce que** le moyen adhésif (19) relie la membrane de dialyse (17) au moins partiellement au cadre.

5. Procédé selon l'une des revendications 1 ou 2, **caractérisé en ce que** le moyen adhésif (19) forme le cadre.

6. Procédé selon l'une des revendications précédentes, **caractérisé en ce que** le filament (18) est un fil, de préférence un fil en nylon.

7. Procédé selon l'une des revendications précédentes, **caractérisé en ce que** le moyen adhésif (19) comprend du cyanoacrylate, du silicone et/ou un époxy.

8. Procédé selon l'une des revendications précédentes, **caractérisé en ce que** le moyen adhésif (19) est un matériau surfacique en colle sensible à la pression, à une ou à deux faces.

9. Procédé selon l'une des revendications précédentes, **caractérisé en ce que**, avant l'introduction de la membrane de dialyse (17) en fibre creuse, une partie de la membrane de dialyse (17) en fibre creuse est entourée par une couche non perméable.
